# EUROPEAN PATENT SPECIFICATION

(11) **EP 3 291 916 B1**
(45) Date of publication and mention of the grant of the patent: **09.09.2020**
(21) Application number: 16790034.9
(22) Date of filing: 04.05.2016
(51) Int. Cl.: B03C 5/00, G01N 27/403, G01N 33/569, G01N 33/543, G01N 33/544

(54) **PARTICLE BASED IMMUNOASSAY WITH ALTERNATING CURRENT ELECTROKINETICS**
PARTIKELBASIERTER IMMUNOASSAY MIT WECHSELSTROMELEKTROKINETIK
DOSAGE IMMUNOLOGIQUE À BASE DE PARTICULES AVEC ÉLECTROCINÉTIQUE EN COURANT ALTERNATIF

(30) Priority: 04.05.2015 US 201562156784 P
(43) Date of publication of application: 14.03.2018
(73) Proprietor: Biological Dynamics, Inc., San Diego, California 92121 (US)
(72) Inventor: CHARLOT, David, San Diego, California 92121 (US); HINESTROSA SALAZAR, Juan Pablo, San Diego, California 92121 (US); THOMAS, George Maroor, San Diego, California 92121 (US); GRIMBERG, Jacob Isaac, San Diego, California 92121 (US); KRISHNAN, Rajaram, San Diego, California 92121 (US)
(74) Representative: Mewburn Ellis LLP
(86) International application number: PCT/US2016/030821
(87) International publication number: WO 2016/179308

(56) References cited:
- WO-A1-2013/112425
- WO-A1-2015/038797
- WO-A2-2009/146143
- US-A1- 2006 228 749
- US-A1- 2012 270 207
- US-A1- 2015 104 858
- R KRISHNAN ET AL: "Alternating current electrokinetic separation and detection of DNA nanoparticles in high-conductance solutions", ELECTROPHORESIS, vol. 29, no. 9, 2008, pages 1765-1774, XP055078619,
- ZHANG Y ET AL: "Individually addressable microelectrode arrays fabricated with gold-coated pencil graphite particles for multiplexed and high sensitive impedance immunoassays", BIOSENSORS AND BIOELECTRONICS, vol. 25, no. 1, 2009, pages 34-40, XP026438579, [retrieved on 2009-06-10]
- SHANSHAN LI ET AL: "Alternating current electrokinetics enhanced in situ capacitive immunoassay", ELECTROPHORESIS, vol. 36, no. 3, 1 February 2015 (2015-02-01), pages 471-474, XP055469947,
- SWAMINATHAN V V ET AL: "Enhanced sub-micron colloidal particle separation with interdigitated microelectrode arrays using mixed AC/DC dielectrophoretic scheme", BIOMEDICAL MICRODEVICES, vol. 17, no. 2, 15 February 2015 (2015-02-15), pages 1-9, XP035499126, [retrieved on 2015-02-15]
- S CHOI ET AL: "Microfluidic-based biosensors toward point-of-care detection of nucleic acids and proteins", MICROFLUIDICS AND NANOFLUIDICS, vol. 10, no. 2, 2010, pages 231-247, XP019876817,
- ÇETIN B ET AL: "Microfluidic bio-particle manipulation for biotechnology", BIOCHEMICAL ENGINEERING JOURNAL, vol. 92, 2014, pages 63-82, XP029077556,

## Description

### BACKGROUND OF THE INVENTION

Biomarker identification based on immunoassays has been expanded greatly in recent years. In addition to gaining novel techniques of diagnosing diseases or identifying disease states, expanded biomarker identification has the potential of adding new tools for monitoring disease progression, treatment efficacy. However, microarray-based methods, including proteomics, gene-based microarrays, imaging techniques and next-generation sequencing, have all generated massive amounts of data that have not translated into clinical practice. Validation of potential biomarkers is especially lacking, where techniques for rapid and efficient clinical assays have not kept pace. In addition, improved sample preparation methods and methods for isolating target markers or other biological material are also lacking. Especially acute where minute sample volumes or amounts are available, the inability to efficiently isolate sample material can hamper downstream biomarker identification efforts.

Zhang Y et al., BIOSENSORS AND BIOELECTRONICS, Vol. 25, No. 1, 2009, pages 34-40 describes individually addressable microelectrode arrays fabricated with gold-coated pencil graphite particles for multiplexed and high sensitive impedance immunoassays.

Krishnan R et al, ELECTROPHORESIS, Vol. 29, No. 9, 2008, pages 1765-1774 describes alternating current electrokinetic separation and detection of DNA nanoparticles in high-conductance solutions.

### SUMMARY OF THE INVENTION

The present invention concerns a method of detecting a target analyte in a sample, comprising,
a. functionalizing a bead in a buffer;
b. contacting the functionalized bead with a primary antibody-labelled conjugate;
c. introducing the functionalized bead-antibody-labelled conjugate into an immunoassay device comprising a sample;
d. introducing a secondary antibody labeled with a fluorescent tag into the device;
e. applying an alternating current (AC) electrokinetic field; and
f. detecting bound analyte,
wherein the immunoassay device is for detecting an analyte in a sample, the device comprising:
(a) a microelectrode array, the array capable of establishing an AC electrokinetic field region and isolating a bead complex in a high conductivity buffer, wherein the bead complex comprises a functionalized bead bound to a labelled-primary antibody;
(b) a fluidic cartridge, the fluidic cartridge capable of housing the microelectrode array and further comprising at least one port for addition and removal of buffers and reagents; and
(c) a fluorescent or luminescent detection system, whereby the presence, absence and/or amount of said analyte in the sample is determined by assessing fluorescence or luminescence from the isolated bead complex bound to a secondary antibody labelled with a fluorescent or luminescent probe.

In one embodiment, the bead is a hydrophilic bead.

In one embodiment, the bead is a polystyrene, poly(methacrylate) or polyacrylate bead.

In one embodiment, the bead is functionalized with streptavidin and the primary antibody is labelled with biotin.

In one embodiment, the bead is functionalized with biotin and the primary antibody is labelled with streptavidin.

In one embodiment, the sample is a bodily fluid, blood, serum, plasma, urine, saliva, a food, a beverage, a growth medium, an environmental sample, a liquid, water, clonal cells, or a combination thereof.

In one embodiment, the fluorescent tag is green fluorescent protein (GFP), cyan fluorescent protein, or yellow fluorescent protein.

In one embodiment, applying the AC electrokinetic field comprises dielectrophoresis.

In one embodiment, applying the AC electrokinetic field creates areas of low and high dielectrophoresis.

In one embodiment, applying the AC electrokinetic field separates analytes by size.

In one embodiment, the analyte is chosen from the group consisting of cellular material, particulate material, cellular particles, exosomes, nucleosomes, liposomes, chromosomes, a protein aggregate, a protein, a peptide, a nucleic acid, fragments thereof and combinations thereof.

In one embodiment, the analyte is an exosome or a nucleosome.

In one embodiment, the analyte is a liposome.

In one embodiment, the analyte is a protein.

In one embodiment, the AC electrokinetic field separates the bound and unbound beads according to charge and size across a platform using dielectrophoresis.

### DESCRIPTION OF THE INVENTION

Disclosed herein are devices and methods for detecting analytes in samples using an immunoassay, the immunoassay using A/C electrokinetics to isolate functionalized bead complexes binding to an analyte.

In one embodiment, disclosed herein are immunoassay devices for detecting an analyte in a sample, the device comprising: (a) a microelectrode array, the array capable of establishing an AC electrokinetic field region and isolating a bead complex in a high conductivity buffer, wherein the bead complex comprises a functionalized bead bound to a labelled-primary antibody; (b) a fluidic cartridge, the fluidic cartridge capable of housing the microelectrode array and further comprising at least one port for addition and removal of buffers and reagents; and (c) a fluorescent or luminescent detection system, whereby the presence, absence and/or amount of said analyte in the sample is determined by assessing fluorescence or luminescence from the isolated bead complex bound to a secondary antibody labelled with a fluorescent or luminescent probe.

In one embodiment, the microelectrode array comprises an array of alternating current (AC) electrodes. In another embodiment, the microelectrode array comprises an array of direct current (DC) electrodes. In yet another embodiment, the microelectrode array is a planar electrode array. In still other embodiments, the AC electrokinetic field is produced using an alternating current having a voltage of 1 volt to 40 volts peak-peak, and/or a frequency of 5 Hz to 5,000,000 Hz and duty cycles from 5% to 50%. In other embodiments, the microelectrode array further comprises a passivation layer with a relative electrical permittivity from about 2.0 to about 4.0. In some embodiments, the conductivity of the fluid is greater than 100 mS/m. In still other embodiments, the microelectrode array is spin-coated with a hydrogel having a thickness between about 0.1 microns to about 1 micron.

In some embodiments, the device disclosed herein include the use of beads, wherein the bead is a hydrophilic bead, a polystyrene bead, a poly(methacrylate) bead or a polyacrylate bead. In some embodiments, the bead is functionalized with streptavidin and the primary antibody is labelled with biotin. In other embodiments, the bead is functionalized with biotin and the primary antibody is labelled with streptavidin. In some embodiments, the fluorescent tag green fluorescent protein (GFP), cyan fluorescent protein, or yellow fluorescent protein. In yet other embodiments, the luminescent tag is luciferin.

In still other embodiments, the sample is a bodily fluid, blood, serum, plasma, urine, saliva, a food, a beverage, a growth medium, an environmental sample, a liquid, water, clonal cells, or a combination thereof.

Also disclosed herein are methods of detecting a target analyte in a sample, the method comprising, a) functionalizing a bead in a buffer; b) contacting the functionalized bead with a primary antibody-labelled conjugate; c) introducing the functionalized bead-antibody-labelled conjugate into a device comprising a sample; d) introducing a secondary antibody labeled with a fluorescent tag into the device; e) applying an alternating current (AC) electrokinetic field; and f) detecting bound analyte.

In some embodiments, the bead employed in the methods disclosed herein is a hydrophilic bead. In other embodiments, the bead is a polystyrene, poly(methacrylate) or polyacrylate bead. In still other embodiments, the bead is functionalized with streptavidin and the primary antibody is labelled with biotin. In yet other embodiments, the bead is functionalized with biotin and the primary antibody is labelled with streptavidin. wherein the fluorescent tag is green fluorescent protein (GFP), cyan fluorescent protein, or yellow fluorescent protein.

In other embodiments, the methods disclosed herein apply an AC electrokinetic field, wherein the AC electrokinetic field comprises dielectrophoresis. In some embodiments, the AC electrokinetic field creates areas of low and high dielectrophoresis. In still other embodiments, the AC electrokinetic field separates analytes by size. In yet other embodiments, the analyte is a protein.

In other embodiments, the AC electrokinetic field separates the bound and unbound beads according to charge and size across a platform using dielectrophoresis.

In still other embodiments, the methods disclosed herein employ fluidic samples, wherein the fludic sample is a bodily fluid, blood, serum, plasma, urine, saliva, a food, a beverage, a growth medium, an environmental sample, a liquid, water, clonal cells, or a combination thereof.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

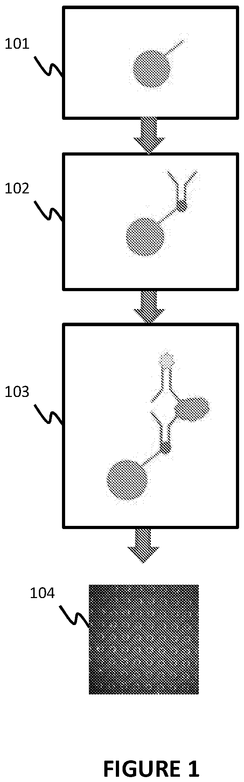

FIG. 1 depicts an example of a procedure of an ACE immunoassay.

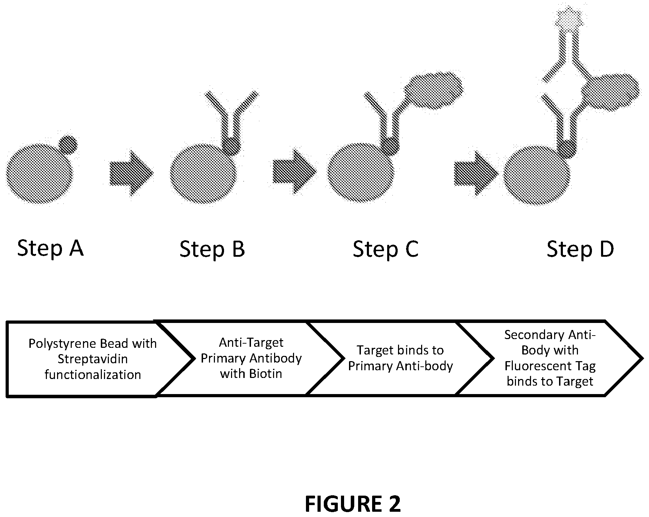

FIG. 2 depicts an example of an incubation process for ACE immunoassay. In step A, polystyrene bead (size 20 to 1000 nm) is functionalized with streptavidin. In step B, an antibody for the protein marker of interest is attached to the bead using streptavidin-biotin binding. In step C, the protein marker of interest is bound to the antibody. In step D, a second antibody modified with a fluorophore is bond to the protein marker of interest.

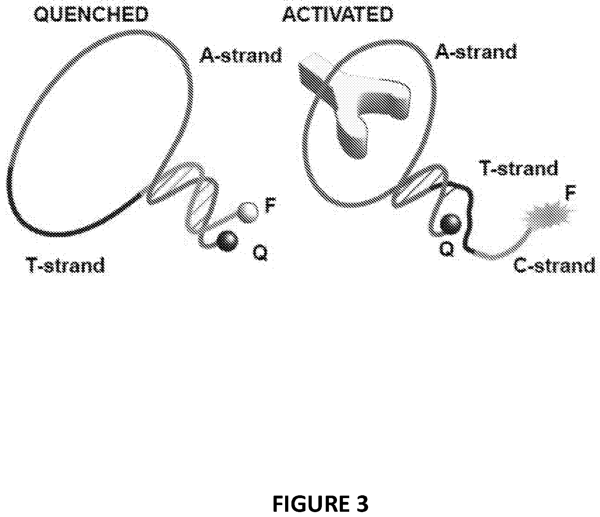

**FIG. 3** depicts an example of a fluorescence resonance energy transfer (FRET) effect.

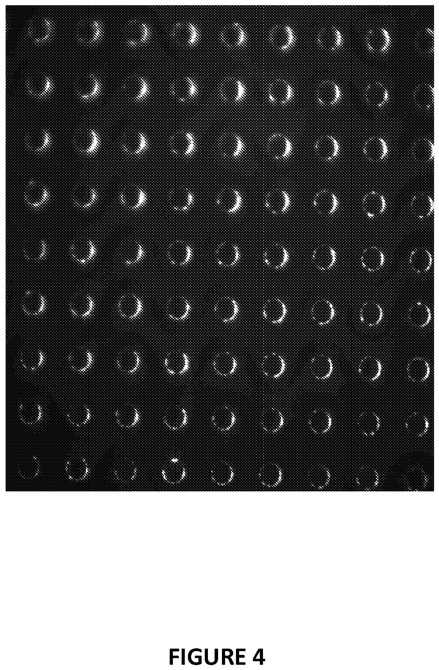

**FIG. 4** Fluorescence (FTIC) microscopy image of a bead-antibody-CEA protein complex captured using the ACE system.

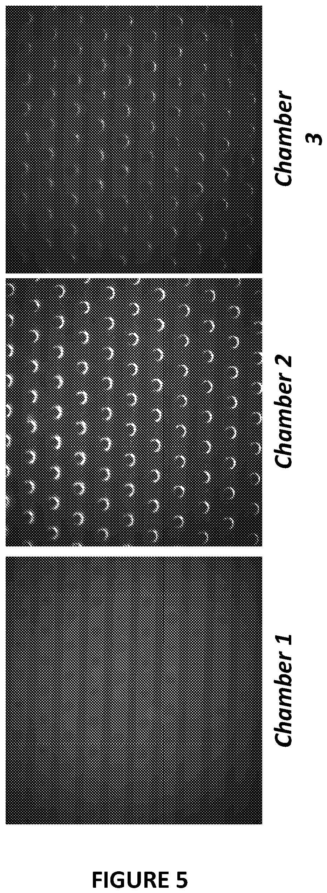

**FIG. 5** **ACE Protein Immunoassay. Chamber 1:** Fluorescein conjugated to Biotin only (-); **Chamber 2:** Fluorescein conjugated to Biotin with unconjugated polystyrene Bead (+); **Chamber 3:** Fluorescein conjugated to Biotin with conjugated polystyrene Bead (-). All images are normalized to 150-40000 absolute fluorescent units (AFU).

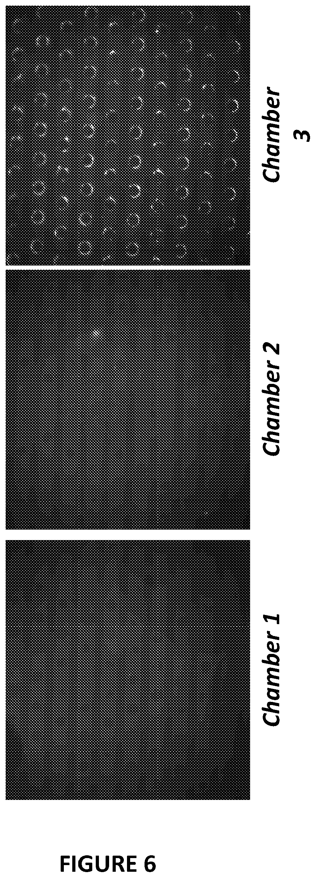

**FIG. 6** ACE Protein Immunoassay; demonstration of immuno-histological capture on ACE system. **Chamber 1:** Anti-IgG-FITC goat antibody; **Chamber 2:** Anti-IgG-FITC goat antibody + unconjugated polystyrene bead; **Chamber 3:** Anti-IgG-FITC goat antibody + polystyrene bead conjugated with anti-CEA antibody.

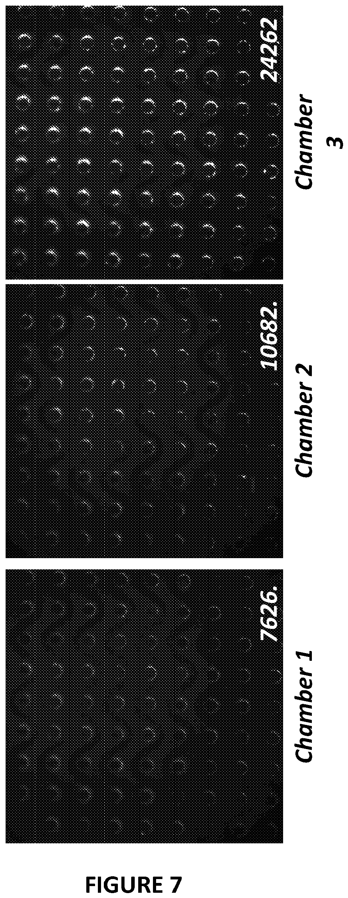

**FIG. 7** ACE Protein Immunoassay. **Chamber 1:** unconjugated Bead+ CEA protein (3800 ng/mL) + Secondary Antibody; **Chamber 2:** conjugated Bead + Secondary Antibody; and **Chamber 3:** conjugated Bead + CEA (3800 ng/mL) + Secondary Antibody. FITC gain: 3; exposure time was 100ms; and all images are normalized to 150-40000.

### DETAILED DESCRIPTION OF THE INVENTION

Described herein are methods, devices and systems suitable for performing immunoassays on samples incorporating A/C (alternating current) electrokinetic (ACE) manipulation and separation of analytes and complexes on an electrode format. In specific embodiments, provided herein are methods, devices and systems for performing immunoassays on a sample by isolating or separating an analyte from a sample comprising other particulate material and performing the immunoassay using ACE manipulation. In some aspects, the methods, devices and systems may allow for rapid analysis of analytes in a sample using immunoassays. In other aspects, the methods, devices and systems may allow for rapid immunoassay analysis of analytes using ACE. In various aspects, the methods, devices and systems may allow for a rapid procedure that requires a minimal amount of material isolated from fluids, including but not limited to biological and environmental samples to rapidly perform immunoassay analysis using ACE.

### Alternating Current Electrokinetic (ACE) Platform and Devices

In some embodiments, described methods, systems, immunoassays, and devices include an ACE platform for isolating, purifying and collecting an analyte from a sample. In one aspect, described herein is an ACE platform for identifying, detecting, quantitating, isolating, purifying and collecting or eluting an analyte from a sample or particulate material, including cells and the like. In other aspects, the platform disclosed herein is capable of identifying, detecting, quantitating, isolating, purifying, collecting and/or eluting analytes from a sample comprising cellular material, particulate material, cellular particles such as exosomes or a molecular construct, including but not limited to nucleosomes, liposomes, chromosomes or a protein aggregate, a protein, a peptide, a nucleic acid, fragments thereof and combinations thereof. In yet other aspects, the platform disclosed herein is capable of identifying, detecting, quantitating, isolating, purifying, collecting and/or eluting analytes from samples comprising a complex mixture of organic and inorganic materials. In some aspects, the platform disclosed herein is capable of identifying, detecting, quantitating, isolating, purifying, collecting and/or eluting analytes from samples comprising organic materials. In yet other aspects, the platform disclosed herein is capable of identifying, detecting, quantitating, isolating, purifying, collecting and/or eluting analytes from samples comprising inorganic materials. In yet other aspects, the platform disclosed herein is capable of identifying one or more mutations in a protein, a peptide, a nucleic acid, fragments thereof and combinations thereof.

In some embodiments, disclosed herein includes an ACE platform for isolating an analyte in a sample, the platform comprising: (1) a heater and/or a reservoir; and (2) a plurality of alternating current (AC) electrodes, the AC electrodes configured to be selectively energized to establish AC electrokinetic high field and AC electrokinetic low field regions, wherein the electrodes comprise conductive material configured on or around the electrodes which reduces, disrupts or alters fluid flow around or within the vicinity of the electrodes as compared to fluid flow in regions between or substantially beyond the electrode vicinity. In certain embodiments, an electrode is a floating electrode as described herein.

In some embodiments, an AC electrokinetic field is generated to collect, separate or isolate analytes for processing in an immunoassay. In some embodiments, the analytes are biomolecules. In some embodiments, the analytes are cellular material, particulate material, a protein, a peptide, a nucleic acid, fragments thereof and combinations thereof In some embodiments, the AC electrokinetic field is a dielectrophoretic field. Accordingly, in some embodiments dielectrophoresis (DEP) is utilized in various steps of the methods, systems, immunoassays, and devices described herein.

Accordingly provided herein includes an ACE platform comprising a plurality of alternating current (AC) electrodes as disclosed herein, the AC electrodes configured to be selectively energized to establish a dielectrophoretic (DEP) field region. In some aspects, the AC electrodes may be configured to be selectively energized to establish multiple dielectrophoretic (DEP) field regions, including dielectrophoretic (DEP) high field and dielectrophoretic (DEP) low field regions. In some instances, AC electrokinetic effects provide for concentration of larger particulate material in low field regions and/or concentration (or collection or isolation) of analytes in high field regions of the DEP field. For example, further description of the electrodes and the concentration of cells in DEP fields may be found in PCT patent publication WO 2009/146143 A2. Further description of floating electrodes that may be used in a device described herein are described in more detail below.

In specific embodiments, DEP is used to concentrate analytes and larger particulate matter either concurrently or at different times. In certain embodiments, methods, systems, immunoassays, and devices described herein are capable of energizing an array of electrodes as disclosed herein so as to produce at least one DEP field. In other embodiments, the methods, systems, immunoassays, and devices described here further comprise energizing the array of electrodes so as to produce a first, second, and any further optional DEP fields. In some embodiments, the methods, systems, immunoassays, and devices described herein are capable of being energized so as to produce a first, second, and any further optional DEP fields.

DEP is a phenomenon in which a force is exerted on a dielectric particle when it is subjected to a non-uniform electric field. Depending on the step of the methods described herein, aspects of the devices and systems described herein, and the like, the dielectric particle in various embodiments herein is a biological analyte. Different steps of the methods described herein or aspects of the systems, immunoassays, and devices described herein may be utilized to isolate and separate different components, such as intact cells or other particulate material; further, different field regions of the DEP field may be used in different steps of the methods or aspects of the devices and systems described herein. The dielectrophoretic force generated in the methods, systems, immunoassays, and devices does not require the particle to be charged. In some instances, the strength of the force depends on the medium and the specific particles' electrical properties, on the particles' shape and size, as well as on the frequency of the electric field. In some instances, fields of a particular frequency selectively manipulate particles. In certain aspects described herein, these processes allow for the separation of analytes from other components, such as cells, cellular debris and proteinaceous material.

Also provided herein are methods, systems, immunoassays, and devices comprising a plurality of direct current (DC) electrodes. In some embodiments, the plurality of DC electrodes comprises at least two rectangular electrodes, spread throughout the array. In some embodiments, the electrodes are located at the edges of the array. In some embodiments, DC electrodes are interspersed between AC electrodes.

In some embodiments, disclosed herein are methods, systems, immunoassays, and devices comprising: (1) a plurality of alternating current (AC) electrodes as disclosed herein, the AC electrodes configured to be selectively energized to establish AC electrokinetic high field and AC electrokinetic low field regions; and (2) a module capable of performing enzymatic reactions, such as polymerase chain reaction (PCR) or other enzymatic reaction. In some embodiments, the plurality of electrodes is configured to be selectively energized to establish a dielectrophoretic high field and dielectrophoretic low field regions. In some embodiments, the methods, systems, immunoassays, and devices are capable of isolating an analyte from a sample, collecting or eluting the analyte and further performing an enzymatic reaction on the analyte. In some embodiments, the enzymatic reaction is performed in the same chamber as the isolation and elution stages. In other embodiments, the enzymatic reaction is performed in another chamber than the isolation and elution stages. In still other embodiments, an analyte is isolated and the enzymatic reaction is performed in multiple chambers.

In some embodiments, the methods, systems, immunoassays, and devices further comprise at least one of an elution tube, a chamber and a reservoir to perform an enzymatic reaction. In some embodiments, the enzymatic reaction is performed in a serpentine microchannel comprising a plurality of temperature zones. In some embodiments, the enzymatic reaction is performed in aqueous droplets entrapped in immiscible fluids (*e.g.,* digital PCR). In some embodiments, the thermal reaction comprises convection. In some embodiments, the device comprises a surface contacting or proximal to the electrodes, wherein the surface is functionalized with biological ligands that are capable of selectively capturing biomolecules.

In one aspect, described herein is an ACE platform comprising electrodes, wherein the electrodes are placed into separate chambers and DEP fields are created within an inner chamber by passage through pore structures. The exemplary systems, immunoassays, and devices include a plurality of electrodes and electrode-containing chambers within a housing. A controller of the device independently controls the electrodes, as described further in PCT patent publication WO 2009/146143 A2.

In some embodiments, chambered ACE platforms are created with a variety of pore and/or hole structures (nanoscale, microscale and even macroscale) and contain membranes, gels or filtering materials which control, confine or prevent cells, particles or other entities from diffusing or being transported into the inner chambers while the AC/DC electric fields, solute molecules, buffer and other small or targeted molecules can pass through the chambers.

Such platforms include, but are not limited to, multiplexed electrode and chambered devices, devices that allow reconfigurable electric field patterns to be created, devices that combine DC electrophoretic and fluidic processes; sample preparation devices, sample preparation, enzymatic manipulation of isolated molecules and diagnostic devices that include subsequent detection and analysis, lab-on-chip devices, point-of-care and other clinical diagnostic systems or versions.

In some embodiments, an ACE platform with a planar electrode array comprises a housing through which a sample fluid flows. In some embodiments, fluid flows from an inlet end to an outlet end, optionally comprising a lateral analyte outlet. The exemplary device includes multiple AC electrodes. In some embodiments, the sample consists of a combination of micron-sized entities or cells, larger analytes and smaller analytes or biomolecules.

In some embodiments, the smaller analytes are cellular material, a protein, a peptide fragment, a nucleic acid, or a combination thereof. In some embodiments, the larger analytes are cellular particles such as exosomes or a molecular construct, including but not limited to nucleosomes, liposomes, chromosomes or a protein aggregate. In some embodiments, the planar electrode array device is a 60x20 electrode array that is optionally sectioned into three 20x20 arrays that can be separately controlled but operated simultaneously. The optional auxiliary DC electrodes can be switched on to positive charge, while the optional DC electrodes are switched on to negative charge for electrophoretic purposes. In some instances, each of the controlled AC and DC systems is used in both a continuous and/or pulsed manner (*e.g*., each can be pulsed on and off at relatively short time intervals) in various embodiments. The optional planar electrode arrays along the sides of the sample flow are optionally used to generate DC electrophoretic forces as well as AC DEP. Additionally, microelectrophoretic separation processes may be optionally carried out, in combination with nanopore or hydrogel layers on the electrode array, using planar electrodes in the array and/or auxiliary electrodes in the x-y-z dimensions.

In various embodiments, the ACE platforms are operated in the AC frequency range of from 1,000 Hz to 100 MHz, at voltages which could range from approximately 1 volt to 2000 volts pk-pk; at DC voltages from 1 volt to 1000 volts, at flow rates of from 10 microliters per minute to 10 milliliter per minute, and in temperature ranges from 1 °C to 120 °C. In some embodiments, the methods, devices and systems are operated in AC frequency ranges of from about 3 to about 15 kHz. In some embodiments, the methods, devices, and systems are operated at voltages of from 5-25 volts pk-pk. In some embodiments, the ACE platforms are operated at voltages of from about 1 to about 50 volts/cm. In some embodiments, the ACE platforms are operated at DC voltages of from about 1 to about 5 volts. In some embodiments, the ACE platforms are operated at a flow rate of from about 10 microliters to about 500 microliters per minute. In some embodiments, the ACE platforms are operated in temperature ranges of from about 20 °C to about 60 °C.

In some embodiments, the ACE platforms are operated in AC frequency ranges of from 1,000 Hz to 10 MHz. In some embodiments, the ACE platforms are operated in AC frequency ranges of from 1,000 Hz to 1 MHz. In some embodiments, the ACE platforms are operated in AC frequency ranges of from 1,000 Hz to 100 kHz. In some embodiments, the ACE platforms are operated in AC frequency ranges of from 1,000 Hz to 10 kHz. In some embodiments, the ACE platforms are operated in AC frequency ranges of from 10 kHz to 100 kHz. In some embodiments, the ACE platforms are operated in AC frequency ranges of from 100 kHz to 1 MHz.

In some embodiments, the ACE platforms are operated at voltages from approximately 1 volt to 1500 volts pk-pk. In some embodiments, the ACE platforms are operated at voltages from approximately 1 volt to 1500 volts pk-pk. In some embodiments, the ACE platforms are operated at voltages from approximately 1 volt to 1000 volts pk-pk. In some embodiments, the ACE platforms are operated at voltages from approximately 1 volt to 500 volts pk-pk. In some embodiments, the ACE platforms are operated at voltages from approximately 1 volt to 250 volts pk-pk. In some embodiments, the ACE platforms are operated at voltages from approximately 1 volt to 100 volts pk-pk. In some embodiments, the ACE platforms are operated at voltages from approximately 1 volt to 50 volts pk-pk.

In some embodiments, the ACE platforms are operated at DC voltages from 1 volt to 1000 volts. In some embodiments, the ACE platforms are operated at DC voltages from 1 volt to 500 volts. In some embodiments, the ACE platforms are operated at DC voltages from 1 volt to 250 volts. In some embodiments, the ACE platforms are operated at DC voltages from 1 volt to 100 volts. In some embodiments, the ACE platforms are operated at DC voltages from 1 volt to 50 volts.

In some embodiments, the AC electrokinetic field is produced using an alternating current having a voltage of 1 volt to 40 volts peak-peak, and/or a frequency of 5 Hz to 5,000,000 Hz and duty cycles from 5% to 50%.

In some embodiments, the ACE platforms are operated at flow rates of from 10 microliters per minute to 1 ml per minute. In some embodiments, the ACE platforms are operated at flow rates of from 10 microliters per minute to 500 microliters per minute. In some embodiments, the ACE platforms are operated at flow rates of from 10 microliters per minute to 250 microliters per minute. In some embodiments, the ACE platforms are operated at flow rates of from 10 microliters per minute to 100 microliters per minute.

In some embodiments, the ACE platforms are operated in temperature ranges from 1 °C to 100 °C. In some embodiments, the ACE platforms are operated in temperature ranges from 20 °C to 95 °C. In some embodiments, the ACE platforms are operated in temperature ranges from 25 °C to 100 °C. In some embodiments, the ACE platforms are operated at room temperature.

In some embodiments, the controller independently controls each of the electrodes. In some embodiments, the controller is externally connected to the device such as by a socket and plug connection, or is integrated with the device housing.

In some embodiments, an ACE platform comprises a housing and a heater or thermal source and/or a reservoir. In some embodiments, the heater or thermal source is capable of increasing the temperature of the fluid to a desired temperature. In some embodiments, the heater or thermal source is suitable for operation as a PCR thermocycler. In other embodiments, the heater or thermal source is used to maintain a constant temperature (isothermal conditions).

In some embodiments, the ACE platform comprises a second reservoir comprising an eluant. The eluant is any fluid suitable for eluting the isolated analyte from the device. In some instances the eluant is water or a buffer.

In some embodiments, an ACE platform described herein is capable of maintaining a constant temperature. In some embodiments, an ACE platform described herein is capable of cooling the electrode array or chamber. In some embodiments, an ACE platform described herein is capable of heating the electrode array or chamber. In some embodiments, an ACE platform described herein comprises a thermocycler. In some embodiments, an ACE platform disclosed herein comprises a localized temperature control element. In some embodiments, an ACE platform disclosed herein is capable of both sensing and controlling temperature.

In some embodiments, an ACE platform further comprises heating or thermal elements. In some embodiments, a heating or thermal element is localized underneath an electrode. In some embodiments, the heating or thermal elements comprise a metal. In some embodiments, the heating or thermal elements comprise tantalum, aluminum, tungsten, or a combination thereof. Generally, the temperature achieved by a heating or thermal element is proportional to the current running through it. In some embodiments, the ACE platforms disclosed herein comprise localized cooling elements. In some embodiments, heat resistant elements are placed directly under the exposed electrode array. In some embodiments, the ACE platforms disclosed herein are capable of achieving and maintaining a temperature between about 20 °C and about 120 °C. In some embodiments, the ACE platforms disclosed herein are capable of achieving and maintaining a temperature between about 30 °C and about 100 °C. In other embodiments, the ACE platforms disclosed herein are capable of achieving and maintaining a temperature between about 20 °C and about 95 °C. In some embodiments, the ACE platforms disclosed herein are capable of achieving and maintaining a temperature between about 25 °C and about 90 °C, between about 25 °C and about 85 °C, between about 25 °C and about 75 °C, between about 25 °C and about 65 °C or between about 25 °C and about 55 °C. In some embodiments, the ACE platforms disclosed herein are capable of achieving and maintaining a temperature of about 20 °C, about 30 °C, about 40 °C, about 50 °C, about 60 °C, about 70 °C, about 80 °C, about 90 °C, about 100 °C, about 110 °C or about 120 °C.

### Electrode designs

New microelectrode array designs have been developed in order to increase the gradient of the electric field generated while also reducing the AC Electrothermal flow generated at any particular voltage. The new designs may comprise a 'floating electrode', *i.e.,* an electrode surrounding the working electrode by not being energized during ACE.

### Samples

The term "biological samples" used herein refers to: biological samples obtained from animals, including humans, such as blood, blood plasma, tissue slices, body fluids, cerebrospinal fluid and urine samples; cells, such as animal, plant, and insect cells; microorganisms, such as bacteria, fungi, and algae; and viruses, including virus-infected cells, although the biological samples are not particularly limited thereto. The term "biological samples" also refers to culture solutions in which such cells, microorganisms, and viruses have been cultured and suspensions of such cells, microorganisms, or viruses. The biological samples include biological molecules that are the targets of separation, extraction, or purification implemented by the sample processing device. The term "biological molecules" used herein refers to proteins (including, but not limited to, cell markers, enzymes or antibodies, peptide fragments, nucleic acids, and cellular particles such as exosomes or a molecular construct, including but not limited to nucleosomes, liposomes, chromosomes or a protein aggregate. The targets of separation, extraction, or purification implemented by the sample processing device are not limited to proteins, and peptide fragments, and compounds produced from cells or microorganisms, including organic compounds or low-molecular-weight compounds, can be subjected to separation, extraction, and purification as biological molecules. Cells include, for example, prokaryotic and eukaryotic cells. Cells may also include bacterial cells. Viruses are also encompassed within the term "biological samples". The term "nucleic acid" as used herein refers to, for example DNA (deoxyribonucleic acid), RNA (ribonucleic acid), and combinations thereof. In some instances, the nucleic acid contains one or more mutations that can be detected using the methods described herein.

Samples can be lysed to detect nucleic acids. Methods of lysing cells are known in the art and are contemplated herein. Other methods of isolating nucleic acids are described, for example, in U.S. Patent No. 8,603,791, issued December 10, 2013.

In some embodiments, the methods described herein free nucleic acids from a plurality of cells by lysing the cells. In some embodiments, nucleic acids are freed from a plurality of cells by lysing the cells.

Samples may be processed as needed prior to use in an immunoassay described herein. For example, blood may be centrifuged to remove plasma, heparin added to prevent clotting, and plasma stored.

In one aspect, the methods described herein can be used to detect an isolated analyte in a sample. In another aspect, the methods described herein can be used to quantitate an analyte in a sample. In yet another aspect, the methods described herein can be used to isolate an analyte from a sample. In some embodiments, the sample comprises a fluid. In one aspect, the sample comprises cells or other particulate material and the analytes. In some embodiments, the sample does not comprise cells.

In some embodiments, the sample is a liquid, optionally water or an aqueous solution or dispersion. In some embodiments, the sample is a bodily fluid. Exemplary bodily fluids include blood, serum, plasma, bile, milk, cerebrospinal fluid, gastric juice, ejaculate, mucus, peritoneal fluid, saliva, sweat, tears, urine, and the like. In some embodiments, analytes are isolated from bodily fluids using the methods, systems or devices described herein as part of a medical therapeutic or diagnostic procedure, device or system. In some embodiments, the sample is tissues and/or cells solubilized and/or dispersed in a fluid medium. For example, the tissue can be a cancerous tumor from which analytes can be isolated using the methods, devices or systems described herein.

In some embodiments, the sample is an environmental sample. In some embodiments, the environmental sample is assayed or monitored for the presence of a particulate or molecule indicative of a certain contamination, infestation incidence or the like. The environmental sample can also be used to determine the source of a certain contamination, infestation incidence or the like using the methods, devices or systems described herein. Exemplary environmental samples include municipal wastewater, industrial wastewater, water or fluid used in or produced as a result of various manufacturing processes, lakes, rivers, oceans, aquifers, ground water, storm water, plants or portions of plants, animals or portions of animals, insects, municipal water supplies, and the like.

In some embodiments, the sample is a food or beverage. The food or beverage can be assayed or monitored for the presence of a particulate or analyte indicative of a certain contamination, infestation incidence or the like. The food or beverage can also be used to determine the source of a certain contamination, infestation incidence or the like using the methods, devices or systems described herein. In various embodiments, the methods, devices and systems described herein can be used with one or more of bodily fluids, environmental samples, and foods and beverages to monitor public health or respond to adverse public health incidences.

In some embodiments, the sample is a growth medium. The growth medium can be any medium suitable for culturing cells, for example lysogeny broth (LB) for culturing *E. coli,* Ham's tissue culture medium for culturing mammalian cells, and the like. The medium can be a rich medium, minimal medium, selective medium, and the like. In some embodiments, the medium comprises or consists essentially of a plurality of clonal cells. In some embodiments, the medium comprises a mixture of at least two species.

In some embodiments, the sample is water, or includes water or any other appropriate buffer needed to process the sample for use in the described methods.

In some embodiments, the sample may also comprise other particulate material. Such particulate material may be, for example, inclusion bodies (*e.g.,* ceroids or Mallory bodies), cellular casts (*e.g*., granular casts, hyaline casts, cellular casts, waxy casts and pseudo casts), Pick's bodies, Lewy bodies, fibrillary tangles, fibril formations, cellular debris and other particulate material. In some embodiments, particulate material is an aggregated protein (*e.g*., beta-amyloid). In yet other embodiments, the sample may comprise other cellular particulate material such as exosomes or a molecular construct, including but not limited to nucleosomes, liposomes, chromosomes or a protein aggregate.

In some embodiments, the fluid is a small volume of liquid including less than about 10 ml. In some embodiments, the fluid is less than about 8 ml. In some embodiments, the fluid is less than about 5 ml. In some embodiments, the fluid is less than about 2 ml. In some embodiments, the fluid is less than about 1 ml. In some embodiments, the fluid is less than about 750 µl. In some embodiments, the fluid is less than about 500 µl. In some embodiments, the fluid is less than about 250 µl. In some embodiments, the fluid is less than about 200 µl. In some embodiments, the fluid is less than about 100 µl. In some embodiments, the fluid is less than about 75 µl. In some embodiments, the fluid is less than about 50 µl. In some embodiments, the fluid is less than about 25 µl. In some embodiments, the fluid is less than about 10 µl. In some embodiments, the fluid is less than about 5 µl. In some embodiments, the fluid is less than about 1 µl.

In some embodiments, the quantity of fluid applied to the device or used in the method comprises less than about 100,000,000 cells. In some embodiments, the fluid comprises less than about 75,000,000 cells. In some embodiments, the fluid comprises less than about 50,000,000 cells. In some embodiments, the fluid comprises less than about 25,000,000 cells. In some embodiments, the fluid comprises less than about 10,000,000 cells. In some embodiments, the fluid comprises less than about 7,500,000 cells. In some embodiments, the fluid comprises less than about 5,000,000 cells. In some embodiments, the fluid comprises less than about 2,500,000 cells. In some embodiments, the fluid comprises less than about 1,000,000 cells. In some embodiments, the fluid comprises less than about 750,000 cells. In some embodiments, the fluid comprises less than about 500,000 cells. In some embodiments, the fluid comprises less than about 250,000 cells. In some embodiments, the fluid comprises less than about 100,000 cells. In some embodiments, the fluid comprises less than about 75,000 cells. In some embodiments, the fluid comprises less than about 50,000 cells. In some embodiments, the fluid comprises less than about 25,000 cells. In some embodiments, the fluid comprises less than about 10,000 cells. In some embodiments, the fluid comprises less than about 7,500 cells. In some embodiments, the fluid comprises less than about 5,000 cells. In some embodiments, the fluid comprises less than about 2,500 cells. In some embodiments, the fluid comprises less than about 1,000 cells.

In some embodiments, isolation of an analyte from a sample with the devices, systems and methods described herein takes less than about 30 minutes, less than about 20 minutes, less than about 15 minutes, less than about 10 minutes, less than about 5 minutes or less than about 1 minute. In other embodiments, isolation of an analyte from a sample with the devices, systems and methods described herein takes not more than 30 minutes, not more than about 20 minutes, not more than about 15 minutes, not more than about 10 minutes, not more than about 5 minutes, not more than about 2 minutes or not more than about 1 minute. In additional embodiments, isolation of an analyte from a sample with the devices, systems and methods described herein takes less than about 15 minutes, preferably less than about 10 minutes or less than about 5 minutes.

In addition, the samples disclosed herein can be used in immunoassays. For instance, in some embodiments, samples containing antigens (*e.g*., peptides, proteins, carbohydrates, lipids, proteoglycans, glycoproteins, *etc.*) in order to assay for antibodies in a bodily fluid sample by sandwich assay, competitive assay, or other formats. Alternatively, the samples may be addressed with antibodies, in order to detect antigens in a sample by sandwich assay, competitive assay, or other assay formats.

In some instances when the analyte is a protein, the protein contains a mutation in one or more amino acid residues. In some instances, the protein contains one mutation; in other instances, the protein contains two mutations; in other instances, the protein contains three mutations; in other instances, the protein contains four mutations; in other instances, the protein contains five mutations; in other instances, the protein contains six mutations; in other instances, the protein contains seven mutations; in other instances, the protein contains eight mutations; in other instances, the protein contains nine mutations; in other instances, the protein contains ten mutations; and in other instances, the protein contains more than ten mutations. A mutation as described herein can encompass an addition, a deletion, a substitution, or a combination thereof of one or more amino acid residues.

In some embodiments, the sample is a biological sample and has a low conductivity or a high conductivity. In some embodiments, the sample comprises a bodily fluid, blood, serum, plasma, urine, saliva, a food, a beverage, a growth medium, an environmental sample, a liquid, water, clonal cells, or a combination thereof. In some embodiments, the cells comprise clonal cells, pathogen cells, bacteria cells, viruses, plant cells, animal cells, insect cells, and/or combinations thereof.

In some embodiments, the sample may comprise a mixture of cell types. For example, blood comprises red blood cells and white blood cells. Environmental samples comprise many types of cells and other particulate material over a wide range of concentrations. In some embodiments, one cell type (or any number of cell types less than the total number of cell types comprising the sample) may be preferentially concentrated.

One would understand that any analyte may be detected using the methods described herein. Analytes include, in some instances, biological markers which may, in turn, be protein markers. Markers also include, in some instances, viruses or cells. Additional analytes that may be tested in the methods include those described in the section above regarding samples.

Non-limiting examples of markers include, for example, cancer markers and markers of inflammation. While cancer markers and markers of inflammation are exemplified herein, one would understand that the described methods are not limited to the disclosed markers. The immunoassays disclosed herein can be used with other markers, including but not limited to tumor markers, cardiac markers, anemia markers, metabolic markers, kidney markers, diabetes markers, thyroid hormone markers, reproductive hormone markers and combinations thereof.

Protein markers for detection using the methods described herein include, but are not limited to, carcinoembryonic antigen (CEA), CA125, CA27.29, CA15-3, CA19.9, alpha-fetoprotein (αFP), β-human chorionic gonadotropin. (βHCG), glypican-1, CYFRA-21, RNA-based markers and prostate specific antigen (PSA).

Additional cancer markers that may be detecting using the methods described herein include, but are not limited to, BRCA1, BRCA2, CD20, Calcitonin, Calretinin, CD34, CD99MIC 2, CD117, Chromogranin, Cytokeratin (various types), Desmin, Epithelial membrane antigen (EMA), Factor VIII, CD31 FL1, Glial fibrillary acidic protein (GFAP), Gross cystic disease fluid protein (GCDFP-15), HER2/neu, HER3, HMB-45, Human chorionic gonadotropin (hCG), inhibin, keratin (various types), lymphocyte marker, MART-1 (Melan-A), Mesothelin, Myo D1, MUC-1, MUC-16 neuron-specific enolase (NSE), placental alkaline phosphatase (PLAP), leukocyte common antigen (CD45), S100 protein, synaptophysin, thyroglobulin, thyroid transcription factor-1, Tumor M2-PK, and vimentin.

Additional markers of inflammation that may be detecting using the methods described herein include, but are not limited to, Carcinoembryonic antigen (CEA), plasma α-fetoprotein (αFP), β human chorionic gonadotrophin (βHCG), C-reactive protein (CRP), Lysosome granules, Histamine, IFN-gamma, Interleukin (IL)-8, Leukotriene B4, Nitric oxide, Prostaglandins, TNF-α, and IL-1.

Cardiac markers include Creatine Kinase (CKMB), Myoglobin and Troponin 1. Markers for anemia include Ferritin. Metabolic markers include Cortisol (CORT) and Human Growth Hormone (HGH). Kidney markers include Cystatin C (CysC), β₂ Microglobulin (BMG), intact Parathyroid Hormone (iPTH). Diabetes markers include C-peptide, Glycated Homoglobin (HbA1c) and Insulin (IRI). Thyroid hormone markers include Tyroid-Stimulating Hormone (TSH) while reproductive hormone markers include βHCG, Follicle-stimulating hormone (FSH), Luteinizing Hormone II (LH II) and Prolactatin (PRL).

When the analyte is a nucleic acid, the nucleic acid isolated using the methods described herein or capable of being isolated by the devices described herein is high-quality and/or suitable for using directly for analysis, including immuno-based assays, in situ hybridization, aptamer-selective isolation, FRET-based analysis and other assays. In some embodiments, the collected nucleic acid comprises at most 0.01 % protein. In some embodiments, the collected nucleic acid comprises at most 0.5% protein. In some embodiments, the collected nucleic acid comprises at most 0.1 % protein. In some embodiments, the collected nucleic acid comprises at most 1 % protein. In some embodiments, the collected nucleic acid comprises at most 2% protein. In some embodiments, the collected nucleic acid comprises at most 3% protein. In some embodiments, the collected nucleic acid comprises at most 4% protein. In some embodiments, the collected nucleic acid comprises at most 5% protein.

In some instances when the analyte is a nucleic acid, the nucleic acid contains a mutation in one or more nucleotides. In some instances, the nucleic acid contains one mutation; in other instances, the nucleic acid contains two mutations; in other instances, the nucleic acid contains three mutations; in other instances, the nucleic acid contains four mutations; in other instances, the nucleic acid contains five mutations; in other instances, the nucleic acid contains six mutations; in other instances, the nucleic acid contains seven mutations; in other instances, the nucleic acid contains eight mutations; in other instances, the nucleic acid contains nine mutations; in other instances, the nucleic acid contains ten mutations; and in other instances, the nucleic acid contains more than ten mutations. A mutation as described herein can encompass an addition, a deletion, a substitution, or a combination thereof of one or more nucleotides.

In some embodiments, the methods described herein further comprise optionally amplifying an isolated nucleic acid by polymerase chain reaction (PCR). In some embodiments, the PCR reaction is performed on or near the array of electrodes or in the device. In some embodiments, the device or system comprise a heater and/or temperature control mechanisms suitable for thermocycling.

In some embodiments, used in conjunction with traditional fluorometry (ccd, pmt, other optical detector, and optical filters), fold amplification is monitored in real-time or on a timed interval. In certain instances, quantification of final fold amplification is reported via optical detection converted to AFU (arbitrary fluorescence units correlated to analyze doubling) or translated to electrical signal via impedance measurement or other electrochemical sensing.

In some embodiments, the nucleic acid is isolated in a form suitable for sequencing or further manipulation of the nucleic acid, including amplification, ligation or cloning.

### Assay Parameters

The present application represents an improvement over commercially available methods in that the methods provide a magnetic bead assay without the presence of magnets.

There are six factors affecting the behavior of magnetic beads that are necessary to observe and control: (1) viscosity of the buffer, (2) ionic force and pH of the buffer, (3) temperature, (4) magnetic content, (5) bead size, and homogeneity of the magnetic force.

For the presently described approach, the only factor that may affect the behavior of the assay will be the temperature and the bead size. Buffer viscosity and ionic force will be standard during the incubation process and once the beads/antibody complex is spiked into the plasma or serum sample the buffering capacity of such fluid will be able to stabilize the process. Since the assay does not use magnetic beads, there are few complications regarding homogeneity of magnetic fields in a batch or from batch-to-batch.

In contrast, the presently described assay's raw materials have very little, if any, batch-to-batch variation. This includes all buffers, magnetic beads, antibodies, *etc.* The production of stable and predictable polyclonal or monoclonal antibodies would be within the knowledge of practitioners in the field.

Beads to be utilized in the present methods may be of any material including, but not limited to, hydrophilic beads, including but not limited to polystyrene, poly(methacrylate) and polyacrylate beads.

In one embodiment, the beads are functionalized or coated in a highly consistent manner. For example, the temperature, pH, and method of suspension are the same from batch to batch. Beads can be functionalized using methods known in the art.

In another embodiment, the concentration of beads and chemicals is consistently maintained from the first to the last aliquot in a lot.

In yet another embodiment, magnetic separation is validated.

The present inventors have identified how heterogeneities during separation can lead to undesirable and irreversible aggregation and uncontrolled losses in product.

### Immunoassays

Described herein are methods, devices, immunoassays, and systems using the size selectivity provided by an ACE platform to develop and perform protein immunoassays.

**Figure 1** shows some embodiments of ACE protein immunoassays. The ACE based immunoassays comprise using polystyrene beads functionalized with streptavidin, see **101.** In **102,** there is a process of incubating beads with biotin-conjugated Anti-CEA antibody for a time period *(e.g.,* 15 min) at a room temperature. The incubation process includes controlling incubation buffers, bead concentration, and/or antibody concentration. In some embodiments, anti-CEA **103** conjugated with FITC is prepared in the above incubation buffer as well at 10X concentration. After antibody-bead conjugation the complex is added to the target sample along with equivalents of anti-CEA conjugated with FITC and allowed to incubate for a time period *(e.g.,* 15 min) at a room temperature; in this step bead concentration may be 10 ng/µL with 5 µmol of antibody per milligram of bead. In additional embodiments, the sample is then run on ACE (VF mod/Temp Cycling) and imaged periodically; an example is shown as image **104** in **Figure 1****.** Running the ACE platform comprises (1) a 2 min static capture and (2) using 20 min flow at 5µL/min using a wash buffer containing a mixture of electrolytes, surfactants and blockers. Exemplary buffered systems include phosphate buffered-saline (PBS), tris-buffered saline (TBS) and the like; exemplary surfactants include TWEEN 20, sodium dodecyl sulfate (SDS), TRITON X100, and the like.

In addition, fluorescence resonance energy transfer (FRET) can be performed using the principle described in **Figure 3****.** Here steps A and B described in **Figure 2** will be performed and then an aptamer with a fluorescent or luminescent tag and a quencher will be bound to the polystyrene bead and then the aptamer sequence will be able to bind the marker of interest. After such binding event, the quencher will leave the aptamer-bead complex and a fluorescence signal can be detected. The bead-aptamer-marker complex will be captured using ACE and after a fluidic wash quantification of the marker of interest will be possible.

**Figure 3** describes an embodiment with an FRET effect. In the Quenched state the fluorophore (F) is quenched by the quencher (Q). After the protein marker or sequence of interest in bound to the aptamer (Activated state) the fluorophore will become un-quenched and a signal can be detected.

In some embodiments, the use of immunoassays comprises an enzymatic reaction and an incubation process, as described in **Figure 2****.** In step A, polystyrene bead (size 20 to 1000 nm) is functionalized with streptavidin. In step B, an antibody for the protein marker of interest is attached to the bead using streptavidin-biotin binding. In step C, the protein marker of interest is bound to the antibody. In step D, a second antibody modified with a fluorophore is bond to the protein marker of interest.

The incubation process is done in the sample in order to bind a protein marker of interest. After this complex is generated, it can be captured in the electrode array of the ACE platform. After a washing, uncaptured material fluorescence microscopy can be used to quantify the abundance of the protein marker. In some instances, a blocking agent can be used to prevent or decrease non-specific binding. Acceptable blocking agents include, but are not limited to, PLURONICS®, TWEEN®, Albumin, and electrolytes.

As shown in **Figure 2****,** steps A and B will be done in an incubation buffer that allows for the streptavidin-biotin reaction to occur successfully. Then this 'active bead' will be spike into the sample of interest, plasma or serum, and it will bind to the available protein markers in the sample and the antibody bearing the fluorophore will introduce. At this point ACE will be turned on and capture will occur.

In some embodiments, immunoassays disclosed comprise particles. Various particles include metal particles, gold particles, conducting particles, or non-conducting particles. In some embodiments, polystyrene particles comprise a coating with gold.

In other embodiments, provided herein is method of detecting a target analyte in a sample, comprising, functionalizing a bead in a buffer; contacting the functionalized bead with a primary antibody-biotin conjugate; introducing the functionalized bead-antibody-biotin conjugate into a device comprising a sample; introducing a secondary antibody labeled with a fluorescent or luminescent tag into the device; applying an alternating current (AC) electrokinetic field; and detecting bound analyte.

A bead to be used in the methods described herein may be of any suitable material including, but not limited to, hydrophilic beads, polystyrene, poly(methacrylate) and polyacrylate.

Beads can be functionalized using methods known in the art with a compound that is capable of binding to a labeled antibody. A non-limiting example of a compound that may be used in the methods described herein includes, for example, streptavidin-biotin, carboxylate-alcohol binding, carboxylate-halogen binding, carboxylate-amino binding, amino-aldehyde binding, amino-alcohol binding and other functionalization methods that are compatible with the materials disclosed herein. As disclosed herein, the functionalized bead particles disclosed herein are capable of, for example, binding to and isolating cellular particles such as exosomes or a molecular construct, including but not limited to nucleosomes, liposomes, chromosomes or a protein aggregate. As disclosed herein, the exomes or molecular constructs present in the samples used herein bind to or interact with the functionalized beads, and are further isolated on the array surface using A/C electrokinetics.

In such methods, the sample can be, for example, a bodily fluid, blood, serum, plasma, cerebrospinal fluid, urine, saliva, a food, a beverage, a growth medium, an environmental sample, a liquid, water, clonal cells, or a combination thereof.

An analyte detected in such methods can be, for example, a cell, cellular material, a protein, a peptide fragment, a nucleic acid, cell membranes, lipid bilayers, RNA, DNA or combinations thereof. In still other embodiments, the analyte can be, for example, cellular particles such as exosomes or a molecular construct, including but not limited to nucleosomes, liposomes, chromosomes or a protein aggregate. In certain embodiments, an analyte detected in such methods can include one or more mutations.

A fluorescent or luminescent tag to be used in the methods described herein may be any suitable fluorescent or luminescent protein used for labeling nucleic acids (DNA, RNA) and proteins, including, luciferase, horseradish peroxidase, acridine dyes, cyanine dyes, fluorone dyes, oxazine dyes, phenanthridine dyes and rhodamine dyes, which includes but is not limited to, rhodamine, Cy2, Cy3, Cy5 Alexa fluorophores, luciferin, fura-2 dyes, green fluorescent protein (GFP), cyan fluorescent protein, and yellow fluorescent protein. The antibodies disclosed herein can be custom synthesized with a variety of fluorescent tags and fluorophores.

It would be understood that the primary and secondary antibodies to be utilized in the methods described herein specifically bind to an analyte to be detected. The term "specifically binds" means that an antibody bind to an epitope with greater affinity than it binds an unrelated amino acid sequence. In one aspect, such affinity is at least 1-fold greater, at least 2-fold greater, at least 3-fold greater, at least 4-fold greater, at least 5-fold greater, at least 6-fold greater, at least 7-fold greater, at least 8-fold greater, at least 9-fold greater, 10-fold greater, at least 20-fold greater, at least 30-fold greater, at least 40-fold greater, at least 50-fold greater, at least 60-fold greater, at least 70-fold greater, at least 80-fold greater, at least 90-fold greater, at least 100-fold greater, or at least 1000-fold greater than the affinity of the antibody for an unrelated amino acid sequence.

"Epitope" refers to that portion of an antigen or other macromolecule capable of forming a binding interaction with the variable region binding pocket of an antibody.

Depending on the amino acid sequence of the constant domain of their heavy chains, immunoglobulins can be assigned to different classes. There are five major classes of immunoglobulins: IgA, IgD, IgE, IgG, and IgM, and several of these can be further divided into subclasses (isotypes), *e.g.,* IgG1, IgG2, IgG3, IgG4, IgA1, and IgA2. The heavy-chain constant domains (Fc) that correspond to the different classes of immunoglobulins are called α, δ, ε, γ, and µ, respectively. The subunit structures and three-dimensional configurations of different classes of immunoglobulins are well known.

The "light chains" of antibodies (immunoglobulins) from any vertebrate species can be assigned to one of two clearly distinct types, called kappa or ("κ" or "K") and lambda or ("λ"), based on the amino acid sequences of their constant domains.

The terms "antigen-binding portion of an antibody," "antigen-binding fragment," "antigen-binding domain," "antibody fragment" or a "functional fragment of an antibody" are used interchangeably herein to refer to one or more fragments of an antibody that retain the ability to specifically bind to an antigen. Non-limiting examples of antibody fragments included within such terms include, but are not limited to, (i) a Fab fragment, a monovalent fragment consisting of the V_{L}, V_{H}, C_{L} and C_{H1} domains; (ii) a F(ab')₂ fragment, a bivalent fragment containing two Fab fragments linked by a disulfide bridge at the hinge region; (iii) a Fd fragment consisting of the V_{H} and C_{H1} domains; (iv) a Fv fragment containing the V_{L} and V_{H} domains of a single arm of an antibody, (v) a dAb fragment (Ward *et al.,* (1989) Nature 341:544 546), which containing a V_{H} domain; and (vi) an isolated CDR. Additionally included in this definition are "one-half" antibodies comprising a single heavy chain and a single light chain. Other forms of single chain antibodies, such as diabodies are also encompassed herein.

A control antibody to be used in a method described herein does not specifically bind to an analyte to be detected.

The immunoassays described herein may be conducted in a device described herein.

Application of an AC electrokinetic field in the methods described herein comprises dielectrophoresis. Applying the AC electrokinetic field creates areas of low and high dielectrophoresis. This application separates bound analyte by size and bound analyte can be detected and, in some instances, quantified using methods known in the art.

In certain instances, calibrators can be run along with a sample of interest in order to make a direct quantification of the isolated protein marker. The calibrator can be a spiked protein of interest at a fixed concentration in a controlled buffer.

In some instances, it is advantageous that the methods described herein are performed in a short amount of time, the devices are operated in a short amount of time, and the systems are operated in a short amount of time. In some embodiments, the period of time is short with reference to the "procedure time" measured from the time between adding the fluid to the device and obtaining isolated analyte. In some embodiments, the procedure time is less than 3 hours, less than 2 hours, less than 1 hour, less than 30 minutes, less than 20 minutes, less than 10 minutes, or less than 5 minutes.

In another aspect, the period of time is short with reference to the "hands-on time" measured as the cumulative amount of time that a person must attend to the procedure from the time between adding the fluid to the device and obtaining isolated analytes. In some embodiments, the hands-on time is less than 20 minutes, less than 10 minutes, less than 5 minute, less than 1 minute, or less than 30 seconds.

In some embodiments, the methods are operated at flow rates of from 10 microliters per minute to 1 ml per minute. In some embodiments, the methods are operated at flow rates of from 10 microliters per minute to 500 microliters per minute. In some embodiments, the methods are operated at flow rates of from 10 microliters per minute to 250 microliters per minute. In some embodiments, the methods are operated at flow rates of from 10 microliters per minute to 100 microliters per minute.

In some embodiments, the methods are operated in temperature ranges from 1 °C to 100 °C. In some embodiments, the methods are operated in temperature ranges from 20 °C to 95°C. In some embodiments, the methods are operated in temperature ranges from 25 °C to 100 °C. In some embodiments, the methods are operated at room temperature.

The limits of detection of the described methods to detect an analyte using the present methods are on par to other immunoassays based on magnetic beads. The resolution of the assay is based on fluorescence intensity relative units.

Assays that may be utilized for this assessment include, but are not limited to, fluorescence resonance energy transfer (FRET), *in situ* hybridization (ISH), fluorescent *in situ* hybridization (FISH), and *Comparative Genomic Hybridization (CGH).*

### Fluorescence resonance energy transfer (FRET)

FRET is a quantum mechanical phenomenon that occurs between a fluorescence donor (D) and a fluorescence acceptor (A) in close proximity to each other (usually <100 Angstrom of separation) if the emission spectrum of D overlaps with the excitation spectrum of A. The molecules to be tested are labeled with a complementary pair of donor and acceptor fluorophores. While bound closely together by the polypeptide interaction, the fluorescence emitted upon excitation of the donor fluorophore will have a different wavelength than that emitted in response to that excitation wavelength when the polypeptides are not bound, providing for quantification of bound versus unbound polypeptides by measurement of emission intensity at each wavelength. Donor: Acceptor pairs of fluorophores with which to label the polypeptides are well known in the art. Fluorophores which may be utilized include, for example, include green fluorescent protein (GFP), variants of the *A. victoria* GFP known as Cyan FP (CFP, Donor (D)) and Yellow FP (YFP, Acceptor (A)). The GFP variants can be made as fusion proteins with the respective members of the binding pair to serve as D-A pairs in a FRET scheme to measure protein-protein interaction. Vectors for the expression of GFP variants as fusions are known in the art and are contemplated for use herein. The addition of a candidate modulator to the mixture of labeled proteins will result in an inhibition of energy transfer evidenced by, for example, a decrease in YFP fluorescence relative to a sample without the candidate modulator.

In an assay using FRET for the detection of a polypeptide interaction, about a 10% or greater decrease in the intensity of fluorescent emission at the acceptor wavelength in samples containing a candidate modulator, relative to samples without the candidate modulator, indicates that the candidate modulator inhibits the polypeptide interaction.

In some instances, a FRET may be modified using fluorescence quenching to monitor molecular interactions: One molecule in the interacting pair can be labeled with a fluorophore, and the other with a molecule that quenches the fluorescence of the fluorophore when brought into close apposition with it. A change in fluorescence upon excitation is indicative of a change in the association of the molecules tagged with the fluorophore:quencher pair. Generally, an increase in fluorescence of the labeled polypeptide is indicative that the polypeptide bearing the quencher has been displaced. In quenching assays, about a 10% or greater increase in the intensity of fluorescent emission in samples containing a candidate modulator, relative to samples without the candidate modulator, indicates that the candidate modulator inhibits the polypeptide interaction.

### In situ hybridization (ISH)

*In situ* hybridization assays are well known and are generally described in Angerer et al., Methods Enzymol. 152:649-660 (1987) and in Parker & Barnes, (1999) Methods in Molecular Biology, 106:247-283. In an *in situ* hybridization assay, cells, *e.g.,* from a biopsy, are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters (FISH).

### Fluorescence in situ hybridization (FISH)

FISH (fluorescence *in situ* hybridization) uses fluorescent probes that bind to only those parts of a sequence with which they show a high degree of sequence similarity. FISH is an assay known in the art wherein a genetic marker can be localized to a chromosome by hybridization. Typically, to perform FISH, a nucleic acid probe that is fluorescently labeled is hybridized to interphase chromosomes that are prepared on a slide. The presence and location of a hybridizing probe can be visualized by fluorescence microscopy. The probe can also include an enzyme and be used in conjunction with a fluorescent enzyme substrate.

FISH is a cytogenetic technique used to detect and localize specific polynucleotide sequences in cells. For example, FISH can be used to detect DNA sequences on chromosomes. FISH can also be used to detect and localize specific RNAs, e.g., mRNAs, within tissue samples. In FISH uses fluorescent probes that bind to specific nucleotide sequences to which they show a high degree of sequence similarity. Fluorescence microscopy can be used to find out whether and where the fluorescent probes are bound. In addition to detecting specific nucleotide sequences, e.g., translocations, fusion, breaks, duplications and other chromosomal abnormalities, FISH can help define the spatial-temporal patterns of specific gene copy number and/or gene expression within cells and tissues.

### Comparative Genomic Hybridization (CGH)

Comparative Genomic Hybridization (CGH) employs the kinetics *of in situ* hybridization to compare the copy numbers of different DNA or RNA sequences from a sample, or the copy numbers of different DNA or RNA sequences in one sample to the copy numbers of the substantially identical sequences in another sample. In many useful applications of CGH, the DNA or RNA is isolated from a subject cell or cell population. The comparisons can be qualitative or quantitative. Procedures are described that permit determination of the absolute copy numbers of DNA sequences throughout the genome of a cell or cell population if the absolute copy number is known or determined for one or several sequences. The different sequences are discriminated from each other by the different locations of their binding sites when hybridized to a reference genome, usually metaphase chromosomes but in certain cases interphase nuclei. The copy number information originates from comparisons of the intensities of the hybridization signals among the different locations on the reference genome. The methods, techniques and applications of CGH are known, such as described in U.S. Pat. No. 6,335,167, and in U.S. App. Ser. No. 60/804,818.

### Removal of Residual Material

In some embodiments, following isolation of the analytes in a DEP field region, the method includes optionally flushing residual material from the isolated analytes. In some embodiments, the devices or systems described herein are capable of optionally and/or comprising a reservoir comprising a fluid suitable for flushing residual material from the analytes. "Residual material" is anything originally present in the sample, originally present in the cells, added during the procedure, created through any step of the process including but not limited to cells *(e.g.,* intact cells or residual cellular material), and the like. For example, residual material includes intact cells, cell wall fragments, proteins, lipids, carbohydrates, minerals, salts, buffers, plasma, and the like. In some embodiments, a certain amount of analyte is flushed with the residual material.

In some embodiments, the residual material is flushed in any suitable fluid, for example in water, TBE buffer, or the like. In some embodiments, the residual material is flushed with any suitable volume of fluid, flushed for any suitable period of time, flushed with more than one fluid, or any other variation. In some embodiments, the method of flushing residual material is related to the desired level of isolation of the analyte, with higher purity analyte requiring more stringent flushing and/or washing. In other embodiments, the method of flushing residual material is related to the particular starting material and its composition. In some instances, a starting material that is high in lipid requires a flushing procedure that involves a hydrophobic fluid suitable for solubilizing lipids.

In some embodiments, the method described herein is optionally utilized to obtain, isolate, or separate any desired analyte that may be obtained from such a method.

In various embodiments, an isolated or separated analyte is a composition comprising analyte that is free from at least 99% by mass of other materials, free from at least 99% by mass of residual cellular material, free from at least 98% by mass of other materials, free from at least 98% by mass of residual cellular material, free from at least 95% by mass of other materials, free from at least 95% by mass of residual cellular material, free from at least 90% by mass of other materials, free from at least 90% by mass of residual cellular material, free from at least 80% by mass of other materials, free from at least 80% by mass of residual cellular material, free from at least 70% by mass of other materials, free from at least 70% by mass of residual cellular material, free from at least 60% by mass of other materials, free from at least 60% by mass of residual cellular material, free from at least 50% by mass of other materials, free from at least 50% by mass of residual cellular material, free from at least 30% by mass of other materials, free from at least 30% by mass of residual cellular material, free from at least 10% by mass of other materials, free from at least 10% by mass of residual cellular material, free from at least 5% by mass of other materials, or free from at least 5% by mass of residual cellular material.

In various embodiments, the analyte has any suitable purity. For example, if an enzymatic assay requires analyte samples having about 20% residual cellular material, then isolation of the analyte to 80% is suitable. In some embodiments, an isolated nucleic analyte comprises less than about 80%, less than about 70%, less than about 60%, less than about 50%, less than about 40%, less than about 30%, less than about 20%, less than about 10%, less than about 5%, or less than about 2% analyte cellular material and/or protein by mass.

In other embodiments, the isolated analyte comprises greater than about 99%, greater than about 98%, greater than about 95%, greater than about 90%, greater than about 80%, greater than about 70%, greater than about 60%, greater than about 50%, greater than about 40%, greater than about 30%, greater than about 20%, or greater than about 10% analyte by mass.

The analytes are isolated in any suitable form.

In some embodiments, the methods described herein result in an isolated analyte sample that is approximately representative of the analyte of the starting sample. In some embodiments, the devices and systems described herein are capable of isolating an analyte from a sample that is approximately representative of the analyte of the starting sample. That is, the population of analytes collected by the method, or capable of being collected by the device or system, are substantially in proportion to the population of analytes present in the cells in the fluid. In some embodiments, this aspect is advantageous in applications in which the fluid is a complex mixture of many cell types and the practitioner desires an analyte-based procedure for determining the relative populations of the various cell types.

In some embodiments, the analyte isolated by the methods described herein or capable of being isolated has a concentration of at least 0.5 ng/mL. In some embodiments, the analyte isolated by the methods described herein or capable of being isolated has a concentration of at least 1 ng/mL. In some embodiments, the analyte isolated by the methods described herein or capable of being isolated has a concentration of at least 5 ng/mL. In some embodiments, the analyte isolated by the methods described herein or capable of being isolated has a concentration of at least 10 ng/ml.

In some embodiments, about 50 pico-grams of analyte is isolated from a sample comprising about 5,000 cells using the methods, systems or devices described herein. In some embodiments, the methods described herein yield at least 10 pico-grams of analyte from a sample comprising about 5,000 cells. In some embodiments, the methods described herein yield at least 20 pico-grams of analyte from a sample comprising about 5,000 cells. In some embodiments, the methods described herein yield at least 50 pico-grams of analyte from about 5,000 cells. In some embodiments, the methods described herein yield at least 75 pico-grams of analyte from a sample comprising about 5,000 cells. In some embodiments, the methods described herein yield at least 100 pico-grams of analyte from a sample comprising about 5,000 cells. In some embodiments, the methods described herein yield at least 200 pico-grams of analyte from a sample comprising about 5,000 cells. In some embodiments, the methods described herein yield at least 300 pico-grams of analyte from a sample comprising about 5,000 cells. In some embodiments, the methods described herein yield at least 400 pico-grams of analyte from a sample comprising about 5,000 cells. In some embodiments, the methods described herein yield at least 500 pico-grams of analyte from a sample comprising about 5,000 cells. In some embodiments, the methods described herein yield at least 1,000 pico-grams of analyte from a sample comprising about 5,000 cells. In some embodiments, the methods described herein yield at least 10,000 pico-grams of analyte from a sample comprising about 5,000 cells. In some embodiments, the methods described herein yield at least 20,000 pico-grams of analyte from a sample comprising about 5,000 cells. In some embodiments, the methods described herein yield at least 30,000 pico-grams of analyte from a sample comprising about 5,000 cells. In some embodiments, the methods described herein yield at least 40,000 pico-grams of analyte from a sample comprising about 5,000 cells. In some embodiments, the methods described herein yield at least 50,000 pico-grams of analyte from a sample comprising about 5,000 cells.

### Definitions

The articles "a", "an" and "the" are non-limiting. For example, "the method" includes the broadest definition of the meaning of the phrase, which can be more than one method.

### EXAMPLES

The application may be better understood by reference to the following non-limiting examples, which are provided as exemplary embodiments of the application. The following examples are presented in order to more fully illustrate embodiments and should in no way be construed, however, as limiting the broad scope of the application. While certain embodiments of the present application have been shown and described herein, it will be obvious that such embodiments are provided by way of example only. Numerous variations, changes, and substitutions may occur to those skilled in the art without departing from the embodiments; it should be understood that various alternatives to the embodiments described herein may be employed in practicing the methods described herein.

### Example 1: Protein Immunoassay

*Materials:* CrossDown buffer, Streptavidin 0.4 - 0.6 µm Polystyrene Particles (PS-Strep), Human Carcino Embryonic Antigen CEA protein (CEA protein), Anti-human carcino embryonic antigen CEA monoclonal antibody, raised in mouse, conjugated to Biotin (Anti-CEA-Biotin) Anti-human carcino embryonic antigen CEA monoclonal antibody, (clone COL-1) raised in mouse, conjugated to FITC (Anti-CEA-FITC), Potassium hydroxide (KCl), Bovine Serum Albumin (BSA), phosphate buffer solution (PBS), TWEEN20, and Non-DEPC treated water were used as received. CrossDown Buffer (PanReacAppliChem) is a special buffer formulated in order to avoid antibody aggregation, cross reactivity and non-specific binding.

*Methods:* Starting solutions (200 nM) of the Anti-CEA-Biotin and Anti-CEA-FITC are prepared by dilution with CrossDown buffer, **(1)** and **(2)** respectively. Similarly, the PS-Strep particles are diluted 1:20 vol/vol using CrossDown buffer **(3).** Solutions of CEA protein in a serum replicator (40 mM KCl + 80 mg/mL BSA) are prepared at different concentrations (10 ng/mL to 200 ng/mL).

The first reagent in the assay, **Reagent A ,** is prepared by mixing **(1)** and **(3)** in a 1:1 vol/vol ratio, and incubating at room temperature for 15 min. Then **Reagent A** is added to the sample of interest (CEA protein in serum replicator) at a 5.55 % vol. final concentration, i.e. 5 µL of **Reagent A** to 90 µL of sample, and this mixture is incubated at room temperature for 15 min. Subsequently, 5 µL of **(2)** are added and further incubation for 15 min at room temperature is performed. After the complex of **Reagent A** - CEA protein - **(2)** is formed, the solution is loaded into the ACE system, run with a specific set of voltage and frequency parameters, and washed with 0.5x PBS with 0.1% TWEEN®20 solution at 5 µL/min. After washing for 20 min the amount of CEA protein captured in the microelectrode array can be quantified via fluorescence microscopy using the FITC parameters for excitation and emission. Figure 4 shows a fluorescent image of the **Reagent A** - CEA protein - **(2)** complex capture on the microelectrode array.

### Example 2: Aptamer precise sequence identification assay

*Materials:* CrossDown buffer, Streptavidin 0.4 - 0.6 µm Polystyrene Particles (PS-Strep), MUC1 Recombinant Human Protein (MUC1), Anti MUC1 Biotinylated Beacon-Aptamer 5'Cy5CTAACCGTbiotindTTTTTTTTTTTTTTTTTT*CACAGGCTACGGCACGTAGAGCATCACCA TGATCCTGTGT*ACGGTTAGA-BHQ2 (MUC1-Biotin-aptamer), Potassium hydroxide (KCl), Bovine Serum Albumin (BSA), phosphate buffer solution (PBS), and Non-DEPC treated water were used as received.

*Methods:* A starting solution (125 µM) of the MUC1-Biotin-aptamer is prepared by dilution with CrossDown buffer (1). Similarly, the PS-Strep particles are diluted 1:20 vol/vol using CrossDown buffer (2). Solutions of MUC1 in a serum replicator (40 mM KCl + 80 mg/mL BSA) are prepared at different concentrations (1 nM to 100 nM).

The first reagent in the assay, **Reagent A,** is prepared by mixing in a 1:1 vol/vol ratio **of (1)** and (2) and incubating at room temperature for 15 min. Then **Reagent A** is added to the sample of interest (MUC1 in serum replicator) at a 5 % vol. final concentration, i.e. 5 µL of **Reagent A** to 95 µL of sample. After the complex of **Reagent A** - **MUC1** sample is formed by incubating at room temperature for 15 min, the solution is loaded into the ACE system, run with an specific set of voltage and frequency parameters, and washed with 0.5x PBS with 0.1% TWEEN20 solution at 5 µL/min. After washing for 20 min the amount of MUC1 captured in the microelectrode array can be quantified via fluorescence microscopy using the Cy5 parameters for excitation and emission.

### Example 3: ACE Protein Immunoassay

An ACE Protein Immunoassay may be conducted using the following steps:

Use 500 nm Polystyrene Beads functionalized with Biotin.

Incubate beads with Streptavidin-conjugated Anti-CEA antibody for 15 min at room temperature (Incubation buffer: AppliChem® Cross Down Buffer (30% in H₂O); Bead concentration: 100 ng/µL; and Antibody concentration: 5 µmol per milligram of Bead).

Anti-CEA conjugated with FITC is prepared in the above incubation buffer as well at 10X concentration.

After antibody-bead conjugation the complex is added to the target sample along with equivalents of anti-CEA conjugated with FITC and allowed to incubate for 15 min at room temperature. Bead Concentration is 10 ng/µL with 5 µmol of antibody per milligram of bead.

The sample is then run on ACE (VF mod/Temp Cycling) and imaged periodically (2 min static capture; 20 min flow at 5µL/min (0.5X PBS, 0.1% TWEEN® 20)).

As seen in **Figure 5****,** fluorescein conjugated with Biotin of gives off limited background; beads with bound antibodies shows decreased fluorescence compared to unbound beads; and unbound beads incubated with Fluorescein tagged with Biotin shows significant capture profile.

As seen in **Figure 6****,** Chamber 1 and 2 show that anti-Igg results in little to no background; and Chamber 3 shows positive signal of anti-Igg antibody binding to the anti-CEA antibody conjugated to the bead. This assay demonstrates immuno-histological capture on the ACE system.

As seen in **Figure 7****,** Chamber 1 suggests capture of CEA + Secondary Antibody complex; Chamber 2 suggests that there are Primary-Secondary antibody interactions; and Chamber 3 is the positive signal from capture of CEA using polystyrene bead.

Calibrators can be run along with a sample of interest in order to make a direct quantification of the isolated CEA. The calibrator can be spiked CEA protein or another protein of interest at a fixed concentration in a controlled buffer.

### Example 4: Potential Examples / Additional Assays

An ACE Protein Immunoassay may be conducted using the following steps:

Polystyrene Beads functionalized with streptavidin.

Incubate beads with biotin-conjugated Anti-HER2 antibody for 15 min at room temperature with blocking buffer; bead concentration: 100 ng/µL; and antibody concentration: 5 µmol per milligram of bead).

Anti-HER2 conjugated with Cy3 is prepared in the above incubation buffer as well at 10X concentration.

After antibody-bead conjugation the complex is added to the target sample along with equivalents of anti-HER2 conjugated with Cy3 and allowed to incubate for 15 min at room temperature. Bead Concentration is 10 ng/µL with 5 µmol of antibody per milligram of bead.

The sample is then run on ACE (VF mod/Temp Cycling) and imaged periodically (2 min static capture; 20 min flow at 5µL/min (0.5X PBS, 0.1% TWEEN® 20)).

## Claims

1. A method of detecting a target analyte in a sample, comprising,
a. functionalizing a bead in a buffer;
b. contacting the functionalized bead with a primary antibody-labelled conjugate;
c. introducing the functionalized bead-antibody-labelled conjugate into an immunoassay device comprising a sample;
d. introducing a secondary antibody labeled with a fluorescent tag into the device;
e. applying an alternating current (AC) electrokinetic field; and
f. detecting bound analyte,
wherein the immunoassay device is for detecting an analyte in a sample, the device comprising:
(a) a microelectrode array, the array capable of establishing an AC electrokinetic field region and isolating a bead complex in a high conductivity buffer, wherein the bead complex comprises a functionalized bead bound to a labelled-primary antibody;
(b) a fluidic cartridge, the fluidic cartridge capable of housing the microelectrode array and further comprising at least one port for addition and removal of buffers and reagents; and
(c) a fluorescent or luminescent detection system, whereby the presence, absence and/or amount of said analyte in the sample is determined by assessing fluorescence or luminescence from the isolated bead complex bound to a secondary antibody labelled with a fluorescent or luminescent probe.

2. The method of claim 1, wherein the bead is a hydrophilic bead.

3. The method of claim 1, wherein the bead is a polystyrene, poly(methacrylate) or polyacrylate bead.

4. The method of claim 1, wherein the bead is functionalized with streptavidin and the primary antibody is labelled with biotin.

5. The method of claim 1, wherein the bead is functionalized with biotin and the primary antibody is labelled with streptavidin.

6. The method of claim 1, wherein the sample is a bodily fluid, blood, serum, plasma, urine, saliva, a food, a beverage, a growth medium, an environmental sample, a liquid, water, clonal cells, or a combination thereof.

7. The method of claim 1, wherein the fluorescent tag is green fluorescent protein (GFP), cyan fluorescent protein, or yellow fluorescent protein.

8. The method of claim 1, wherein applying the AC electrokinetic field comprises dielectrophoresis.

9. The method of claim 1, wherein applying the AC electrokinetic field creates areas of low and high dielectrophoresis.

10. The method of claim 9, wherein applying the AC electrokinetic field separates analytes by size.

11. The method of claim 1, wherein the analyte is chosen from the group consisting of cellular material, particulate material, cellular particles, exosomes, nucleosomes, liposomes, chromosomes, a protein aggregate, a protein, a peptide, a nucleic acid, fragments thereof and combinations thereof.

12. The method of claim 1, wherein the analyte is an exosome or a nucleosome.

13. The method of claim 1, wherein the analyte is a liposome.

14. The method of claim 1, wherein the analyte is a protein.

15. The method of claim 1, wherein the AC electrokinetic field separates the bound and unbound beads according to charge and size across a platform using dielectrophoresis.

## Patentansprüche

1. Verfahren zum Detektieren eines Target-Analyten in einer Probe, das Folgendes umfasst:
a. das Funktionalisieren eines Kügelchens in einem Puffer;
b. das Inkontaktbringen des funktionalisierten Kügelchens mit einem Primärantikörpermarkierten Konjugat;
c. das Einführen des funktionalisierten Kügelchen-Antikörper-markierten Konjugats in eine Immuntestvorrichtung, die eine Probe umfasst;
d. das Einführen eines sekundären Antikörpers, der mit einer fluoreszierenden Markierung markiert ist, in die Vorrichtung;
e. das Anlegen eines elektrokinetischen Wechselstrom- (AC-) Feldes; und
f. das Detektieren des gebunden Analyten,
wobei die Immuntestvorrichtung zur Detektion eines Analyten in einer Probe vorgesehen ist, wobei die Vorrichtung Folgendes umfasst:
(a) einen Mikroelektrodenarray, wobei der Array in der Lage ist, einen elektrokinetischen Wechselstrom- (AC-) Feldbereich zu erzeugen und einen Kügelchenkomplex in einem Puffer mit hoher Leitfähigkeit zu isolieren, wobei der Kügelchenkomplex ein funktionalisiertes Kügelchen umfasst, das an einen markierten Primärantikörper gebunden ist;
(b) eine Fluidkartusche, wobei die Fluidkartusche in der Lage ist, den Mikroelektrodenarray einzuhausen, und außerdem zumindest eine Öffnung zum Zusetzen und Entfernen von Puffern und Reagenzien aufweist; und
(c) ein Fluoreszenz- oder Lumineszenz-Detektionssystem, wobei das Vorhandensein, Nichtvorhandensein und/oder die Menge des Analyten in der Probe bestimmt wird, indem die Fluoreszenz oder Lumineszenz von dem isolierten Kügelchenkomplex, der an einen mit einer fluoreszierenden oder lumineszierenden Sonde markierten sekundären Antikörper gebunden ist, gemessen wird.

2. Verfahren nach Anspruch 1, wobei das Kügelchen ein hydrophiles Kügelchen ist.

3. Verfahren nach Anspruch 1, wobei das Kügelchen ein Polystyrol-, Poly(methacrylat)- oder Polyacrylatkügelchen ist.

4. Verfahren nach Anspruch 1, wobei das Kügelchen mit Streptavidin funktionalisiert ist und der Primärantikörper mit Biotin markiert ist.

5. Verfahren nach Anspruch 1, wobei das Kügelchen mit Biotin funktionalisiert ist und der Primärantikörper mit Streptavidin markiert ist.

6. Verfahren nach Anspruch 1, wobei die Probe ein Körperfluid, Blut, Serum, Plasma, Urin, Speichel, ein Nahrungsmittel, ein Getränk, ein Wachstumsmedium, eine Umweltprobe, eine Flüssigkeit, Wasser, klonale Zellen oder eine Kombination davon ist.

7. Verfahren nach Anspruch 1, wobei die fluoreszierende Markierung grün fluoreszierendes Protein (GFP), cyanfarben fluoreszierendes Protein oder gelb fluoreszierendes Protein ist.

8. Verfahren nach Anspruch 1, wobei das Anlegen des elektrokinetischen AC-Feldes Dielektrophorese umfasst.

9. Verfahren nach Anspruch 1, wobei das Anlegen des elektrokinetischen AC-Feldes Bereiche mit niedriger und hoher Dielektrophorese erzeugt.

10. Verfahren nach Anspruch 9, wobei das Anlegen des elektrokinetischen AC-Feldes die Analyten nach Größe trennt.

11. Verfahren nach Anspruch 1, wobei der Analyt aus der aus Zellmaterial, Teilchenmaterial, Zellpartikeln, Exosomen, Nucleosomen, Liposomen, Chromosomen, einem Proteinaggregat, einem Protein, einem Peptid, einer Nucleinsäure, Fragmenten davon und Kombinationen davon bestehenden Gruppe ausgewählt ist.

12. Verfahren nach Anspruch 1, wobei der Analyt ein Exosom oder ein Nucleosom ist.

13. Verfahren nach Anspruch 1, wobei der Analyt ein Liposom ist.

14. Verfahren nach Anspruch 1, wobei der Analyt ein Protein ist.

15. Verfahren nach Anspruch 1, wobei das elektrokinetische AC-Feld die gebundenen und ungebundenen Kügelchen nach Ladung und Größe auf einer Plattform mittels Dielektrophorese trennt.

## Revendications

1. Procédé de détection d'un analyte cible dans un échantillon, comprenant les étapes consistant à :
a. fonctionnaliser une bille dans un tampon ;
b. mettre en contact la bille fonctionnalisée avec un conjugué marqué par un anticorps primaire ;
c. introduire le conjugué marqué par un anticorps-bille fonctionnalisée dans un dispositif de dosage immunologique comprenant un échantillon ;
d. introduire un anticorps secondaire marqué avec une étiquette fluorescente dans le dispositif ;
e. appliquer un champ électrocinétique à courant alternatif (CA) ; et
f. détecter l'analyte lié,
dans lequel le dispositif de dosage immunologique sert à détecter un analyte dans un échantillon, le dispositif comprenant :
(a) un réseau de microélectrodes, le réseau étant capable d'établir une région de champ électrocinétique CA et d'isoler un complexe de bille dans un tampon à conductivité élevée, dans lequel le complexe de bille comprend une bille fonctionnalisée liée à un anticorps primaire marqué ;
(b) une cartouche fluidique, la cartouche fluidique étant capable de loger le réseau de microélectrodes et comprenant en outre au moins un orifice pour l'addition et le retrait de tampons et de réactifs ; et
(c) un système de détection par fluorescence ou luminescence, dans lequel la présence, l'absence et/ou la quantité dudit analyte dans l'échantillon est déterminée en évaluant la fluorescence ou la luminescence du complexe de bille isolé lié à un anticorps secondaire marqué avec une sonde fluorescente ou luminescente.

2. Procédé selon la revendication 1, dans lequel la bille est une bille hydrophile.

3. Procédé selon la revendication 1, dans lequel la bille est une bille de polystyrène, de poly(méthacrylate) ou de polyacrylate.

4. Procédé selon la revendication 1, dans lequel la bille est fonctionnalisée avec de la streptavidine et l'anticorps primaire est marqué avec de la biotine.

5. Procédé selon la revendication 1, dans lequel la bille est fonctionnalisée avec de la biotine et l'anticorps primaire est marqué avec de la streptavidine.

6. Procédé selon la revendication 1, dans lequel l'échantillon est un fluide corporel, du sang, du sérum, du plasma, de l'urine, de la salive, un aliment, une boisson, un milieu de croissance, un échantillon environnemental, un liquide, de l'eau, des cellules clonales ou une combinaison de ceux-ci.

7. Procédé selon la revendication 1, dans lequel l'étiquette fluorescente est une protéine fluorescente verte (GFP), une protéine fluorescente cyan ou une protéine fluorescente jaune.

8. Procédé selon la revendication 1, dans lequel l'application du champ électrocinétique CA comprend une diélectrophorèse.

9. Procédé selon la revendication 1, dans lequel l'application du champ électrocinétique CA comprend des zones de diélectrophorèse basse et élevée.

10. Procédé selon la revendication 9, dans lequel l'application du champ électrocinétique CA sépare des analytes par taille.

11. Procédé selon la revendication 1, dans lequel l'analyte est choisi dans le groupe comprenant un matériau cellulaire, un matériau particulaire, des particules cellulaires, des exosomes, des nucléosomes, des liposomes, des chromosomes, un agrégat de protéines, une protéine, un peptide, un acide nucléique, des fragments de ceux-ci et des combinaisons de ceux-ci.

12. Procédé selon la revendication 1, dans lequel l'analyte est un exosome ou un nucléosome.

13. Procédé selon la revendication 1, dans lequel l'analyte est un liposome.

14. Procédé selon la revendication 1, dans lequel l'analyte est une protéine.

15. Procédé selon la revendication 1, dans lequel le champ électrocinétique CA sépare les billes liées et non liées selon la charge et la taille à travers une plate-forme en utilisant une diélectrophorèse.
